# FASCICULE DE BREVET EUROPEEN

(11) **EP 2 161 075 B1**
(45) Date de publication et mention de la délivrance du brevet: **25.07.2012**
(21) Numéro de dépôt: 09167939.9
(22) Date de dépôt: 14.08.2009
(51) Int. Cl.: B01J 2/16, B01J 2/30, C07C 51/41, C07C 227/38, C07C 229/76, C07F 1/00, C07F 3/00, C07F 11/00, C07F 15/02, C07F 15/06

(54) **Procédé et dispositif pour la fabrication de complexes organometalliques en poudre**
Verfahren und Vorrichtung zur Herstellung von Organometallische Komplexe in pulver form
Process and device for manufacturing organometallic complexes in powder form

(30) Priorité: 26.08.2008 FR 0855713
(43) Date de publication de la demande: 10.03.2010
(73) Titulaire: Pancosma S.A., 1218 Le Grand-Saconnex (CH)
(72) Inventeur: Meunier, Jean-Philippe, 01170 CESSY (FR); Oguey, Sébastien, 1128 REVEROLLE (CH)
(74) Mandataire: Poncet, Jean-François

(56) Documents cités:
- EP-A- 0 256 645
- WO-A-03/049850

## Description

L'invention concerne les complexes organométalliques conditionnés en poudre qui, par leur nature, sont destinés à être employés en tant que source de métal biodisponible pour l'animal ou l'homme. Ces complexes sont généralement des combinaisons d'acides aminés et de métaux, et sont désignés par les appellations chélates ou complexes.

Exposés à l'air humide, de tels complexes en poudre sont susceptibles de prendre rapidement l'humidité. Inversement, exposés à des élévations de température, ils sont susceptibles de perdre de l'eau. Ce faisant, ils deviennent durs, prennent en masse, forment une croûte ou même deviennent pâteux ou liquides. Ce phénomène est appelé mottage ou déliquescence. Il en résulte que les complexes ne sont alors plus hydrosolubles et parfaitement dispersibles dans le même temps.

Ces inconvénients perturbent fortement les utilisations essentielles de ces complexes pour l'alimentation animale, dans lesquelles on veut pouvoir mélanger de façon homogène le complexe en dose réduite dans un substrat, par exemple en dose de l'ordre de 1 à 1 000 grammes par tonne, ou dans des mélanges en dose de l'ordre de 1 à 250 ‰.

Il faut en effet disposer d'une poudre suffisamment fine, et dont la finesse reste suffisante pendant le mélange avec le substrat. Or dans les conditions d'usage habituelles, le substrat contient fréquemment une certaine quantité d'eau libre. Le mélange n'est alors possible que si la poudre présente une hygroscopicité suffisamment faible pour que ses grains restent détachés et dispersibles malgré la présence de l'humidité.

L'invention concerne en particulier les complexes organométalliques d'acides aminés hydrosolubles tels que la glycine, la valine, la thréonine, la lysine,...

A ce jour un nombre restreint de techniques sont employées pour obtenir des produits en poudre, dont la plus courante est le séchage par atomisation, encore appelé "spray dry".

Le séchage par atomisation consiste à amener la solution aqueuse concentrée contenant le produit à sécher dans une turbine ou des buses de manière à pulvériser cette solution en fines gouttelettes dans une chambre à haute température (entre 150 et 220°C). Là, l'eau est immédiatement évaporée, et les très fines particules de produit sont transportées par un courant d'air jusque dans un cyclone pour séparer l'air humide et le produit sec.

La technologie de séchage par atomisation produit nécessairement des gouttelettes pulvérisées de très petite taille, à très faible teneur en eau, et amorphes. Elle ne permet pas de sélectionner efficacement le taux d'humidité et la granulométrie du produit fini. Le produit obtenu présente une hygroscopicité très importante, ce qui entraîne les phénomènes de mottage ou de déliquescence.

L'invention vise à résoudre ce problème en conférant au complexe une forme physique et/ou chimique appropriée présentant en elle-même une hygroscopie nettement plus réduite que les composés similaires obtenus au moyen des procédés habituellement utilisés, sans nuire au caractère hydrosoluble. En pratique, l'invention propose un complexe organométallique cristallin dans lequel l'acide aminé est lié au métal par au moins un oxygène de son groupe carbonyle.

Pour réaliser des glycinates de zinc, de cuivre, de fer ou de manganèse en poudre cristalline, le document WO 2003/049850 a récemment recommandé l'utilisation du séchage sous vide avec brassage dans une installation de séchage sous vide, tout en dissuadant d'utiliser toutes les autres technologies (atomisation, lit fluidisé...). Il est apparu en effet possible de réaliser des poudres cristallines, en contrôlant, par cette technique, le temps de séchage. Toutefois, l'expérience a montré que le séchage sous vide présente plusieurs inconvénients :
- le temps de fabrication pour un lot est long, conduisant à des coûts élevés ;
- l'utilisation de sécheurs de très grande taille n'apporte pas de gain de productivité conséquent ;
- le procédé de fabrication est très sensible, la moindre variation d'un des paramètres de séchage (température, pression, agitation,...) entraîne une modification de la qualité du produit fini et change le temps de séchage ;
- le procédé de fabrication nécessite de grandes compétences techniques pour permettre de garder une qualité constante.

Il en résulte que la qualité des poudres obtenues industriellement par un tel procédé n'est pas constante et reproductible.

Le problème proposé par la présente invention est d'éviter les inconvénients des procédés connus, en proposant un nouveau procédé permettant de produire des complexes organométalliques d'acide aminé hydrosolubles, parfaitement dispersibles et présentant une grande stabilité dans les conditions habituelles d'usage.

On cherche en particulier à ce que le procédé donne un résultat suffisamment constant et reproductible.

L'invention vise à résoudre ce problème sans adjonction de produits supplémentaires.

L'idée qui est à la base de l'invention est d'utiliser la technique du lit fluidisé, contrairement à la dissuasion exprimée dans le document WO 2003/049850, et d'adapter des moyens permettant de maîtriser la granulation et la cristallisation du complexe organométallique.

Pour atteindre ces buts ainsi que d'autres, l'invention propose notamment un procédé de production d'un complexe organométallique cristallin en poudre, comprenant les étapes de :
a) préparer une solution aqueuse du complexe organométallique,
b) granuler et sécher ladite solution par pulvérisation dans un lit fluidisé par circulation d'air,
c) extraire du lit fluidisé les grains de poudre lorsque ceux-ci ont atteint une taille supérieure à une taille limite basse prédéterminée et un taux d'humidité inférieur à un taux d'humidité maximum prédéterminé.

Les propriétés de stabilité des poudres obtenues dépendent alors essentiellement de leur granulométrie à l'issue du procédé, le taux d'humidité pouvant varier dans des proportions assez larges, soit de 2 % à 20 % en poids sans que cela nuise à la stabilité.

Le processus de granulation peut être réalisé par un système fonctionnant par lot (discontinu) ou en continu. Pour ces deux systèmes, un certain nombre de paramètres doivent êtres maîtrisés afin d'obtenir un produit final dont les propriétés sont constantes.

En priorité, il est important d'obtenir une taille satisfaisante.

Selon une possibilité, ladite taille limite basse prédéterminée est de 25 µm environ. Cela permet d'obtenir une stabilité satisfaisante vis-à-vis de l'hygrométrie ambiante, et une adaptation à des applications telles que l'aquaculture, l'alimentation des animaux domestiques ou de compagnie.

Selon une autre possibilité, ladite taille limite basse est de 80 µm environ. Cela permet d'obtenir une stabilité satisfaisante vis-à-vis de l'hygrométrie ambiante dans les applications à l'alimentation des animaux d'élevage tels que les bovins.

De préférence, la taille des grains est une taille comprise entre une taille limite basse prédéterminée et une taille limite haute prédéterminée. On favorise ainsi une meilleure homogénéité des lots produits et de leurs propriétés, et on évite la présence de grains trop gros pour les applications à l'alimentation animale.

En pratique, pendant l'étape de granulation et de séchage dans un lit fluidisé, on choisit les paramètres de température et de débit de la solution aqueuse et de l'air de façon à obtenir, en fin d'étape, des grains de poudre ayant un taux d'humidité prédéterminé.

Pour cela on détermine des températures d'entrée et de sortie d'air, le flux (Kg/min) de la solution aqueuse à sécher afin de fabriquer un produit présentant un taux d'humidité prédéterminé et une structure physique et chimique désirée.

Le taux d'humidité maximum prédéterminé peut avantageusement être compris entre 2 % et 20 % en poids.

Selon un premier mode de réalisation, le procédé est continu.

Dans ce cas, :
- on granule et on sèche en continu ladite solution par pulvérisation dans un lit fluidisé par air,
- on adapte en permanence les paramètres de température et de débit de la solution aqueuse et de l'air de façon à obtenir dans le lit fluidisé un taux d'humidité inférieur à un taux d'humidité maximum prédéterminé,
- on extrait en continu du lit fluidisé les grains de poudre ayant une taille supérieure à une taille limite basse prédéterminée.

Au cours d'une étape préalable d'apprentissage, on peut déterminer des flux et températures d'air d'entrée, d'air de sortie, et de solution aqueuse d'entrée, ces flux et températures permettant de produire un flux de grains de poudre en sortie présentant un taux d'humidité choisi qui, mesuré par prélèvement d'échantillons, est inférieur au taux d'humidité maximum prédéterminé. Il suffit ensuite de maintenir lesdites valeurs de flux et de températures pendant la durée du procédé, pour obtenir, en sortie, des poudres présentant la structure physique et chimique désirée, et les propriétés désirées.

Selon un second mode de réalisation, le procédé est discontinu, et on traite en un seul lot une masse de matière. Dans ce cas :
- on granule et on sèche un lot de matière par pulvérisation de ladite solution dans un lit fluidisé par air,
- après que les grains de poudre dans le lit fluidisé ont atteint une taille supérieure à la taille limite basse prédéterminée, on interrompt la pulvérisation en poursuivant le maintien en lit fluidisé pour réaliser un séchage jusqu'à atteindre un taux d'humidité inférieur au taux d'humidité maximum prédéterminé,
- on extrait la masse de grains de poudre ayant ces taille et taux d'humidité.

La première étape du procédé, selon laquelle on prépare une solution aqueuse du complexe organométallique, permet la réalisation du complexe lui-même, par une réaction de complexation.

Pour cela, pendant cette étape, on peut avantageusement maintenir une température du mélange dans la plage de 55°C à 80°C.

De préférence, pendant cette étape de préparation de solution aqueuse du complexe organométallique, on peut mélanger en chauffant, dans un mélangeur, de l'eau, de l'acide aminé et un sel métallique hydrosoluble, jusqu'à atteindre une température de 80°C, que l'on maintient pendant une durée suffisante, supérieure à 15 minutes, pour la réaction de complexation.

En pratique, il est préférable d'introduire l'eau et de la chauffer en premier, dans le mélangeur, puis d'ajouter l'acide aminé et le sel métallique en quantité équimolaire, et on agite et on chauffe le mélange jusqu'à atteindre une température de 55°C à 80°C, de préférence 80°C, pendant au moins 15 minutes suivant le métal considéré.

Selon une première application du procédé, pour réaliser du glycinate de fer anhydre, on utilise comme acide aminé la glycine, on utilise comme sel métallique du sulfate de fer Il heptahydrate, et le taux d'humidité est d'environ 6 %.

Selon une seconde application, pour réaliser du glycinate de cuivre dihydrate, on utilise comme acide aminé la glycine, on utilise comme sel métallique du sulfate de cuivre pentahydrate, et le taux d'humidité est d'environ 13 %.

Selon une autre application, pour réaliser du glycinate de zinc anhydre, on utilise comme acide aminé la glycine, on utilise comme sel métallique du sulfate de zinc monohydrate, et le taux d'humidité est d'environ 2 %.

Selon une autre application, pour réaliser du glycinate de zinc dihydrate, on utilise comme acide aminé la glycine, on utilise comme sel métallique du sulfate de zinc monohydrate, et le taux d'humidité est d'environ 13 %.

Selon une autre application, pour réaliser du glycinate de zinc trihydrate, on utilise comme acide aminé la glycine, on utilise comme sel métallique du sulfate de zinc monohydrate, et le taux d'humidité est d'environ 17 %.

Selon un autre aspect, l'invention propose un dispositif pour la mise en oeuvre du procédé tel que défini ci-dessus, le dispositif comprenant :
- un mélangeur et des moyens de chauffe et de régulation de température pour produire une solution aqueuse homogène de complexe organométallique à température appropriée,
- un système de pulvérisation en lit fluidisé, avec une enceinte et des moyens de création d'un flux d'air de suspension ascendant dans l'enceinte, et avec des moyens pour recevoir du mélangeur un débit approprié de ladite solution aqueuse de complexe organométallique et pour le pulvériser dans l'enceinte,
- des moyens d'extraction pour extraire du lit fluidisé les grains de poudre ayant une taille satisfaisante et un taux d'humidité satisfaisant.

De préférence, les moyens d'extraction comprennent un dispositif de sélection pour prélever des grains de poudre dans le lit fluidisé, des moyens pour sélectionner et évacuer en sortie parmi les grains de poudre prélevés ceux qui ont une taille satisfaisante, et des moyens pour recycler dans le lit fluidisé les grains de poudre non sélectionnés.

D'autres objets, caractéristiques et avantages de la présente invention ressortiront de la description suivante de modes de réalisation particuliers, faite en relation avec les figures jointes, parmi lesquelles :
- la figure 1 est une vue schématique d'une installation de granulation par lit fluidisé, selon un procédé de traitement par lots ou en continu ;
- la figure 2 illustre schématiquement le fonctionnement d'un lit fluidisé à injection par le haut ;
- la figure 3 illustre schématiquement le fonctionnement d'un lit fluidisé à injection par le bas ;
- la figure 4 est une coupe longitudinale illustrant schématiquement une installation de lit fluidisé en continu à trois chambres de traitement successives ; et
- la figure 5 illustre la structure générale d'une installation de production en continu selon un mode de réalisation de la présente invention.

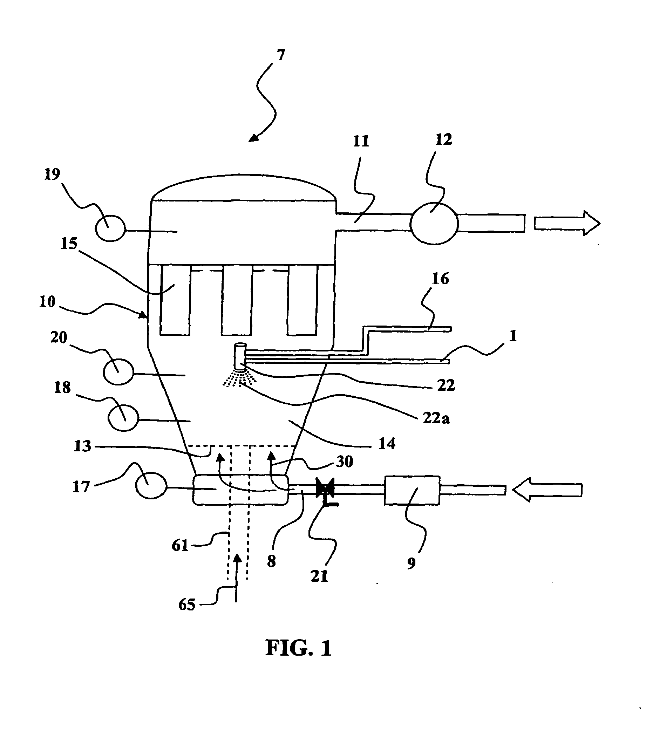

On se réfère maintenant aux figures 1, 2 et 3, pour l'explication de la technique de lit fluidisé.

Le principe du lit fluidisé est basé sur la création d'un flux d'air ascendant de suspension par un phénomène d'aspiration. Ce flux d'air ascendant, dont la pression et la température sont fixées selon des paramètres prédéfinis, est canalisé afin de traverser de bas en haut un lit de matière pulvérulente et d'entraîner sa mise en suspension.

On distingue, sur le système 7 à lit fluidisé selon la figure 1 : une entrée d'air de suspension 8 avec système de filtration et de chauffage 9, laissant entrer de l'air de suspension 30 chaud à la base d'une enceinte 10 ayant une sortie d'air supérieure 11 raccordée à l'atmosphère par un dispositif d'aspiration 12. Une grille de distribution 13, en zone inférieure de l'enceinte 10, limite la base d'une zone de lit fluidisé 14, ou zone de pulvérisation sur des produits pulvérulents. Au-dessus de la zone de lit fluidisé 14 se trouve une zone de filtration 15, en amont de la sortie 11 dans le sens d'écoulement de l'air de suspension.

Des moyens de pulvérisation de liquide sont prévus dans le dispositif pour pulvériser la solution aqueuse de complexe organométallique dans la zone de lit fluidisé 14. Dans la réalisation illustrée sur la figure 1, il s'agit d'une pulvérisation depuis le dessus (top spray). La pulvérisation s'effectue par injection de la solution aqueuse 1 et d'air de pulvérisation 16.

On contrôle la température d'entrée d'air de suspension 30 par des moyens de contrôle de température inférieurs 17, la température dans la zone de lit fluidisé 14 par des moyens de contrôle de température intermédiaires 18, la température de sortie d'air 11 par des moyens de contrôle de température supérieurs 19, et le taux d'humidité dans les grains de poudre dans la zone de lit fluidisé 14 par un dispositif de contrôle d'humidité 20. La quantité d'air de suspension 30 admise est contrôlée par une vanne d'entrée d'air 21.

La buse de pulvérisation 22 est placée au-dessus des particules en suspension dans la zone de lit fluidisé 14, et la pulvérisation 22a de l'agent mouillant s'effectue de haut en bas.

Partant du fond de l'enceinte 10 limité par la grille 13, les particules sont entraînées et accélérées par le courant d'air de suspension 30, passent près de la buse de pulvérisation 22 et reçoivent des gouttelettes de la solution aqueuse de complexe organométallique qui en sortent. Les gouttelettes de solution aqueuse se déposent sur les particules en formant une couche d'enrobage à chacun de leur passage. Cette opération est réalisée à contre-courant par rapport au flux du produit. Les particules subissent un mouvement ascendant traversant la zone de lit fluidisé 14 vers la partie supérieure ou chambre d'expansion de l'enceinte 10. Elles sont ensuite ralenties lorsqu'elles atteignent le haut de la partie d'expansion, puis retombent sur les côtés et sont de nouveau soumises au courant d'air fluidisant. Ce cycle continue pendant toute la durée du procédé.

L'appareil se compose ainsi de deux sections en contact avec le produit :
- une enceinte 10 ayant avantageusement une forme de cuve conique, équipée d'un fond perforé ou grille 13 pour la retenue du produit à traiter, et équipée de la buse de pulvérisation 22 orientée vers le bas, positionnable à deux niveaux de la cuve selon la quantité de produit traité ;
- une extension supérieure qui forme le logement d'un filtre à manche, constituant la zone de filtration 15 qui permet de retenir le produit en traitement pour éviter son échappement par la sortie d'air supérieure 11.

Périodiquement, pendant le procédé, le filtre est décolmaté par secouage mécanique qui permet aux particules fines de retourner dans le lit fluidisé pour poursuite de la granulation.

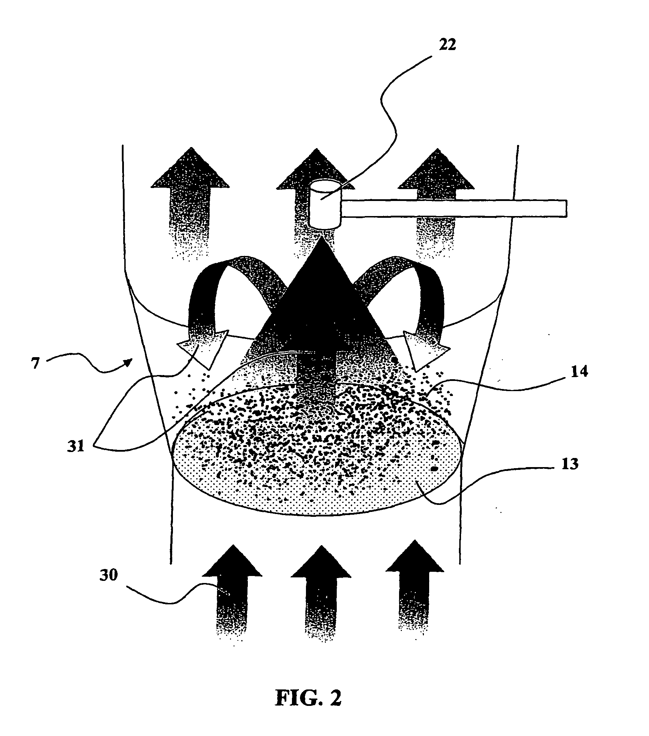

La figure 2 illustre à nouveau, en perspective partielle, le système 7 de la figure 1, en représentant le flux d'air ascendant de suspension 30 et le flux des particules 31 dans la zone de lit fluidisé 14.

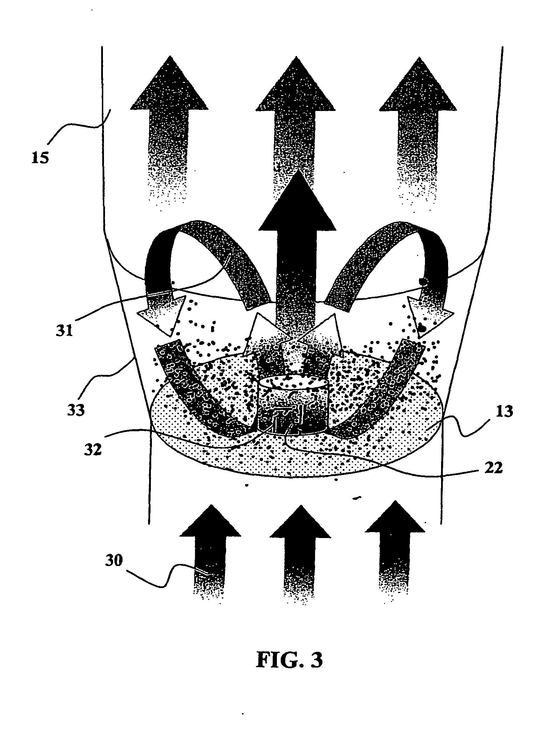

La figure 3 illustre un système de granulation en lit fluidisé à pulvérisation par le bas. La buse de pulvérisation 22 est placée en fond de cuve, au centre de la plaque perforée ou grille 13, et surmontée d'un cylindre 32. La pulvérisation de la solution aqueuse de complexe organométallique s'effectue de bas en haut. L'appareil se compose d'une cuve amovible 33 de type WÜRSTER de forme conique s'adaptant sur le logement du filtre à manche 15 de l'appareil. La cuve 33 comporte une plaque de fond de cuve perforée 13, un cylindre 32 (facultatif) situé dans la partie inférieure de la cuve au-dessus de la plaque perforée et dont la hauteur est réglable, et une buse 22 située au centre de la plaque perforée 13, sous le cylindre 32, et orientée pour une pulvérisation de bas en haut. Le volume d'air plus important qui traverse le centre de la plaque perforée 13 et le cylindre 32 interne créent de ce fait un flux ascendant 31 de matière qui redescend ensuite vers l'extérieur en donnant aux particules en suspension un mouvement dit "en fontaine". La matière en suspension circule rapidement de cette façon et, à chaque passage du produit près de la buse de pulvérisation 22, reçoit une couche d'enrobage liquide supplémentaire. A l'arrêt de la pulvérisation du liquide d'enrobage succède le séchage qui se poursuit dans la même cuve avec simplement une augmentation de la température et du débit de l'air ascendant 30 entrant. On obtient ainsi des grains de matière à surface très régulière.

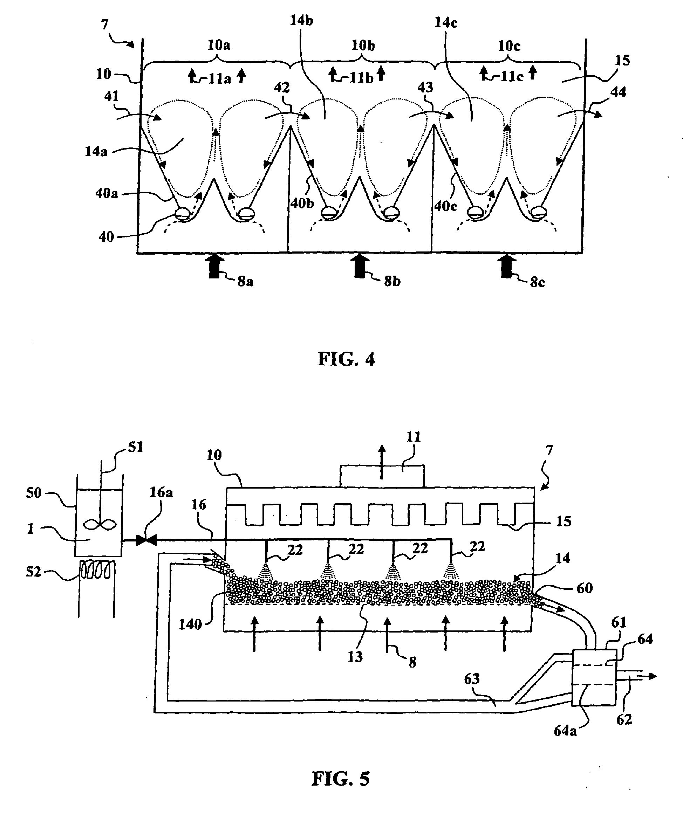

On peut avantageusement utiliser un procédé de lit fluidisé en continu, et non plus par lots. Des exemples sont illustrés schématiquement sur les figures 4 et 5.

Dans le cas de la figure 4, le procédé consiste à fluidiser le lit par un courant d'air qui traverse non pas une grille de fond mais des fentes inférieures longues. La figure 4 illustre le dispositif en coupe transversale, où l'enceinte 10 est divisée par exemple en trois chambres de traitement 10a, 10b et 10c ayant chacune une entrée d'air de suspension respective 8a, 8b ou 8c, une sortie d'air supérieure 11a, 11b ou 11c, et une ou deux fentes longitudinales inférieures telles que la fente 40 pour faire passer l'air dans la zone de lit fluidisé 14a, 14b ou 14c à travers une paroi de séparation inférieure 40a, 40b ou 40c. Les buses de pulvérisation, non illustrées, peuvent pulvériser vers le haut ou vers le bas.

Des moyens sont prévus pour faire transiter, par exemple par gravité, la matière granulaire d'un compartiment à l'autre comme illustré par les flèches 41, 42, 43 et 44, en continu. En pratique, le passage de la matière granulaire d'un compartiment à l'autre peut être réalisé grâce à l'orientation du flux d'air dans l'enceinte 10.

Ce principe peut être appliqué à une enceinte 10 comportant trois chambres de traitement 10a, 10b et 10c comme illustré sur la figure 4, ou à une enceinte 10 comportant un nombre différent de chambres de traitement.

Une description d'un tel dispositif de lit fluidisé en continu à plusieurs chambres peut être trouvée par exemple dans le document US 2006/0228661 A1.

On considère maintenant le dispositif illustré sur la figure 5, permettant la mise en oeuvre du procédé selon la présente invention, et comprenant une seule chambre de traitement 10 en continu.

On retrouve, dans ce dispositif, l'enceinte 10 avec les moyens pour générer et entretenir un lit fluidisé 140 dans une zone de lit fluidisé 14 et par exemple : quatre buses 22 connectées à la canalisation 16, la grille inférieure 13, l'entrée d'air inférieure 8, la sortie d'air supérieure 11.

La canalisation 16 est raccordée à un mélangeur 50 muni de moyens d'agitation 51 et de moyens de chauffe 52. Les moyens de chauffe 52 sont pilotés par des moyens de régulation de température pour assurer l'établissement d'une température appropriée dans le mélangeur 50.

Le dispositif de lit fluidisé 7 à enceinte 10 est un dispositif de type continu. Une vanne 16a permet de régler le débit d'entrée de solution aqueuse provenant du mélangeur 50.

En sortie 60 de l'enceinte 10, on prévoit un dispositif de sélection 61 apte à laisser passer sur sa sortie 62 les grains de poudre présentant une taille comprise entre une limite inférieure et une limite supérieure. Les grains de taille inférieure ou supérieure aux limites respectives sont recyclés dans l'enceinte 10 pour la poursuite de la granulation.

Par exemple, le dispositif de sélection 61 peut comprendre deux tamis 64 et 64a. Les deux tamis laissent passer les particules de petit diamètre vers la canalisation de recyclage 63 pour leur recyclage. Le tamis 64 retient les particules de gros diamètre pour les renvoyer en recyclage, et laisser passer les particules de taille moyenne convenable, qui sont retenues par le tamis 64a pour être dirigées vers la sortie 62.

En considérant maintenant à nouveau la figure 1, on peut également prévoir un dispositif de sélection 61, par exemple sous la forme d'une sortie inférieure centrale dépourvue de tamis mais dans laquelle on fait circuler un flux d'air en contre flux 65 qui opère une sélection en laissant passer les particules de gros diamètre et en repoussant les particules plus fines.

Le procédé de granulation de glycinates a pu être mis en oeuvre avec succès.

Les essais ont permis de déterminer la structure chimique des échantillons et de s'assurer du caractère cristallin des poudres obtenues, en évaluant la stabilité des échantillons à la température et à l'humidité.

La diffraction des rayons X a été utilisée pour s'assurer de la structure chimique et du caractère cristallin des produits obtenus (seuls les produits cristallins diffractent les rayons X).

Le taux d'humidité des poudres obtenues a été déterminé par dessiccation halogène (Mettler-Toledo HR73).

La stabilité à la température a été analysée à l'étuve à 60°C pendant 6 heures dans un pilulier scellé.

La stabilité à l'humidité a été analysée à 30°C et 70 % d'humidité, pendant 6 heures, dans un pilulier ouvert.

On a ainsi produit plusieurs types de glycinate, en choisissant les conditions de température et de débit.

On peut distinguer :
- les glycinates de type I, dont la formule est : [M(SO₄)(C₂H₅NO₂)]ₙ
- les glycinates de type II, dont la formule est : [M(C₂H₅NO₂)(H₂O)₂(SO₄)].
- les glycinates de type III, dont la formule est :

   {[M(C₂H₅NO₂)(H₂O)₂(SO₄)₂] [M(C₂H₅NO₂)(H₂O)₄]}ₙ,
où M est un métal tel que Fer, Zinc ...

### Exemple N°1 : Glycinate de fer par un procédé continu

La synthèse du glycinate de fer peut être opérée comme suit.

On réalise un mélange en poids de 30,5 % d'eau, 56,50 % de sulfate de fer II heptahydrate et 13 % de glycine.

L'eau est chauffée à 60°C, puis le sulfate de fer II heptahydrate et la glycine sont introduits dans l'eau dans un mélangeur.

Le mélange est agité et chauffé jusqu'à ce qu'il atteigne une température de 70°C pendant au moins 15 minutes.

Cette solution est ensuite séchée (par granulation) en lit fluidisé par un système de pulvérisation par le bas « bottom spray » dans un appareil Glatt Procell 5 (Glatt GmbH, Weimar, Allemagne).

Le débit d'air de suspension dans l'appareil Glatt Procell 5 est fixé à 150 m³/heure. La température du produit est de 59°C. La pression d'air de pulvérisation est de 2,5 bars. Le débit de pulvérisation est de 20 g/minute. Le diamètre de la buse est de 1,2 mm. La température d'entrée d'air est de 95°C. La température de sortie d'air est de 50°C.

On prélève en continu le produit ayant une taille de grains satisfaisante. La mesure du taux d'humidité peut être effectuée à l'aide d'un dessiccateur infrarouge ou halogène, ou par thermogravimétrie. Dans le cas présent le taux d'humidité est de 5,6% (formulation 1).

Le produit analysé est cristallin. Il diffracte les rayons X. Il est constitué majoritairement de glycinate de fer anhydre (type I).

L'échantillon fabriqué est parfaitement stable à la température et à l'humidité. En outre, son aspect physique très régulier, avec l'absence quasi totale de particules fines, lui assure une excellente coulabilité et l'absence de pulvérulence.

### Exemple N°2 : Glycinates de cuivre

La synthèse des glycinates de cuivre peut être opérée comme suit.

On réalise un mélange en poids de 37,69 % d'eau, de 48,02 % de sulfate de cuivre pentahydrate et de 14,29 % de glycine.

L'eau est chauffée à 55°C, puis le sulfate de cuivre II pentahydrate et la glycine sont introduits dans l'eau dans un mélangeur.

Le mélange est agité et chauffé jusqu'à ce qu'il atteigne une température de 70°C pendant au moins 15 minutes.

Cette solution est ensuite séchée (par granulation) en lit fluidisé par un système de pulvérisation par le bas « bottom spray » dans un appareil Glatt Procell 5 (Glatt GmbH, Weimar, Allemagne).

Le débit d'air de suspension dans l'appareil Glatt Procell 5 est fixé à 100 m³/heure. La température du produit est de 65°C. La pression d'air de pulvérisation est de 2,5 bars. Le débit de pulvérisation est de 23 g/minute. Le diamètre de la buse est de 1,2 mm. La température d'entrée d'air est de 110°C. La température de sortie d'air est de 50°C.

On prélève en continu le produit ayant une taille de grains satisfaisante. La mesure du taux d'humidité peut être effectuée à l'aide d'un dessiccateur infrarouge ou halogène, ou par thermogravimétrie. Dans le cas présent plusieurs taux d'humidité ont été testés (tableau N°1).

**Tableau N°1 : Taux d'humidité des formulations F2 à F6**

| Echantillon | Taux d'humidité |
|---|---|
| Glycinate de cuivre (Formulation 2) | 13 % |
| Glycinate de cuivre (Formulation 3) | 12 % |
| Glycinate de cuivre (Formulation 4) | 9 % |
| Glycinate de cuivre (Formulation 5) | 7 % |
| Glycinate de cuivre (Formulation 6) | 5 % |

Tous les produits analysés sont cristallins, ils diffractent tous les rayons X.

Le taux d'humidité affecte la structure chimique du glycinate de cuivre (tableau N°2).

**Tableau N°2: Estimations de la proportion en poids de glycinate de cuivre dihydrate (type II) et anhydre (Type I)**

| Echantillon | % GCu dihydrate | % GCu anhydre |
|---|---|---|
| Formulation 2 | 97,9 | 2,1 |
| Formulation 3 | 96 | 4 |
| Formulation 4 | 86 | 14 |
| Formulation 5 | 75 | 25 |
| Formulation 6 | 57 | 43 |

Tous les échantillons testés sont parfaitement stables à la température. En outre, leur aspect physique très régulier, avec l'absence quasi totale de particules fines, leur assure une excellente coulabilité et l'absence de pulvérulence.

### Exemple N° 3 : Glycinates de zinc

La synthèse des glycinates de zinc peut être opérée comme suit.

On réalise un mélange en poids de 57,7 % d'eau, 29,9 % de sulfate de zinc monohydrate, et 12,5 % de glycine.

L'eau est chauffée à 55°C, puis le sulfate de zinc monohydrate et la glycine sont introduits dans l'eau dans un mélangeur.

Le mélange est agité et chauffé jusqu'à ce qu'il atteigne une température de 70°C pendant au moins 15 minutes.

Cette solution est ensuite séchée (granulation) en lit d'air fluidisé par un système de pulvérisation par le bas « bottom spray » dans un appareil Glatt Procell 5 (Glatt GmbH, Weimar, Allemagne).

Le débit d'air de suspension dans l'appareil Glatt Procell 5 est fixé à 150 m³/heure. La température du produit est de 59°C. La pression d'air de pulvérisation est de 2,5 bars. Le débit de pulvérisation est de 23 g/minute. Le diamètre de la buse est de 1,2 mm. La température d'entrée d'air est de 95°C. La température de sortie d'air est de 50°C.

On prélève en continu le produit ayant une taille de grains satisfaisante. La mesure du taux d'humidité peut être effectuée à l'aide d'un dessiccateur infrarouge ou halogène ou par thermogravimétrie. Dans le cas présent plusieurs taux d'humidité ont été testés (tableau N°3).

**Tableau N°3 : Taux d'humidité des formulations F7 à F9**

| Echantillon | Taux d'humidité |
|---|---|
| Glycinate de zinc (formulation 7) | 2 % |
| Glycinate de zinc (formulation 8) | 13 % |
| Glycinate de zinc (formulation 9) | 17 % |

Tous les produits analysés sont cristallins, ils diffractent tous les rayons X.

Le glycinate de zinc de la formulation 7 est constitué de glycinate de zinc anhydre (type I).

Le glycinate de zinc de la formulation 8 est constitué de glycinate de zinc dihydrate (type II).

Le glycinate de zinc de la formulation 9 est constitué de glycinate de zinc trihydrate (type III).

Tous les échantillons testés sont parfaitement stables à la température et à l'humidité. En outre, leur aspect physique très régulier, avec l'absence quasi totale de particules fines, leur assure une excellente coulabilité et l'absence de pulvérulence.

### Exemple N° 4 : Glycinate de fer par un procédé par lot

On prépare une solution aqueuse de glycinate de fer à partir d'eau, de sulfate de fer II heptahydrate et de glycine comme dans l'exemple 1 ci-dessus.

Cette solution est ensuite séchée (par granulation) en lit fluidisé par lot dans un appareil Glatt Procell 5, dans lequel on applique les paramètres ci-après pour un traitement du lot entier de matière contenu dans l'appareil.

Le débit d'air de suspension est fixé à 150 m3/heure. Le débit de pulvérisation est de 25 g/minutes. Le diamètre de la buse est de 1,2 mm. Pendant la période de pulvérisation, la température du produit à l'intérieur de l'appareil est de 55°C. La température d'entrée d'air est de 90°C. La température de sortie d'air est de 46°C. A la fin de l'étape de pulvérisation, le produit est séché en augmentant la température du produit jusqu'à obtention du taux d'humidité souhaité. Dans le cas présent : 6%. Lorsqu'il n'y a plus de pulvérisation de solution dans le système, la température du produit augmente naturellement. Sans faire varier les paramètres d'entrée d'air, il suffit d'attendre que le taux d'humidité descende.

L'échantillon fabriqué est parfaitement stable à la température et à l'humidité. En outre, son aspect physique très régulier, avec l'absence quasi totale de particules fines, lui assure une excellente coulabilité et l'absence de pulvérulence.

En conclusion, la technologie du lit fluidisé permet d'obtenir aisément des complexes cristallins hydrosolubles de la glycine ou des autres acides aminés hydrosolubles tels que la valine, la thréonine, la lysine,...

Selon un aspect, l'invention prévoit ainsi un complexe organométallique cristallin en poudre, dans lequel les grains de poudre ont tous une taille supérieure à 25 µm environ.

La structure cristalline et la granulométrie élevée de tels produits permettent aux produits d'être stables à l'humidité et à la température. Ils sont en outre parfaitement libres d'écoulement et dépourvus de fines particules indésirables.

De préférence, pour les applications aux additifs alimentaires pour l'alimentation des animaux d'élevage, l'invention prévoit un complexe organométallique cristallin en poudre, dans lequel les grains de poudre ont une taille comprise entre 100 et 800 µm environ, avantageusement entre 200 et 400 µm environ.

De la sorte, la granulométrie régulière favorise encore la constance des propriétés de lots successifs de produits, et évite la présence de grains trop gros qui ne seraient pas appropriés pour les applications dans l'alimentation animale.

De préférence, les grains de poudre ont un taux d'humidité inférieur à 20 % environ, avantageusement inférieur à 6 % environ.

Au résultat du procédé de fabrication, les grains de poudre ont une structure de grain issu d'un lit fluidisé, c'est-à-dire qu'ils ont une structure stratifiée compacte.

La présente invention n'est pas limitée aux modes de réalisation qui ont été explicitement décrits, mais elle en inclut les diverses variantes et généralisations contenues dans le domaine des revendications ci-après.

## Revendications

1. Procédé de production d'un complexe organométallique cristallin en poudre à partir d'acides aminés hydrosolubles comprenant les étapes de :
a) préparer une solution aqueuse du complexe organométallique,
b) granuler et sécher ladite solution par pulvérisation dans un lit fluidisé par circulation d'air,
c) extraire du lit fluidisé les grains de poudre lorsque ceux-ci ont atteint une taille supérieure à une taille limite basse prédéterminée et un taux d'humidité inférieur à un taux d'humidité maximum prédéterminé.

2. Procédé selon la revendication 1, **caractérisé en ce que** ladite taille limite basse prédéterminée est de 25 µm environ.

3. Procédé selon la revendication 1, **caractérisé en ce que** ladite taille limite basse prédéterminée est de 80 µm environ.

4. Procédé selon l'une quelconque des revendications 1 à 3, **caractérisé en ce que**, pendant l'étape de granulation et de séchage dans un lit fluidisé, on choisit les paramètres de température et de débit de la solution aqueuse et de l'air de façon à obtenir, en fin d'étape, des grains de poudre ayant un taux d'humidité prédéterminé.

5. Procédé selon l'une quelconque des revendications 1 à 4, **caractérisé en ce que** le taux d'humidité maximum est compris entre 2 % et 20 % en poids.

6. Procédé selon l'une quelconque des revendications 1 à 5, **caractérisé en ce que** :
- on granule et on sèche en continu ladite solution par pulvérisation dans un lit fluidisé par air,
- on adapte en permanence les paramètres de température et de débit de la solution aqueuse et de l'air de façon à obtenir dans le lit fluidisé un taux d'humidité inférieur à un taux d'humidité maximum prédéterminé,
- on extrait en continu du lit fluidisé les grains de poudre ayant une taille supérieure à une taille limite basse prédéterminée.

7. Procédé selon la revendication 6, **caractérisé en ce qu'**au cours d'une étape préalable d'apprentissage, on détermine des flux et températures d'air d'entrée, d'air de sortie et de solution aqueuse d'entrée permettant de produire un flux de grains de poudre en sortie présentant un taux d'humidité choisi, mesuré par prélèvement d'échantillons, inférieur au taux d'humidité maximum prédéterminé, et on maintient ensuite lesdites valeurs de flux et de températures pendant la durée du procédé.

8. Procédé selon l'une quelconque des revendications 1 à 5, **caractérisé en ce que** :
- on granule et on sèche un lot de matière par pulvérisation de ladite solution dans un lit fluidisé par air,
- après que les grains de poudre dans le lit fluidisé ont atteint une taille supérieure à la taille limite basse prédéterminée, on interrompt la pulvérisation en poursuivant le maintien en lit fluidisé pour réaliser un séchage jusqu'à atteindre un taux d'humidité inférieur au taux d'humidité maximum prédéterminé,
- on extrait la masse de grains de poudre ayant ces taille et taux d'humidité.

9. Procédé selon l'une quelconque des revendications 1 à 8, **caractérisé en ce que**, pendant l'étape de préparation du complexe organométallique, on maintient une température du mélange dans la plage de 55°C à 80°C environ.

10. Procédé selon l'une quelconque des revendications 1 à 9, **caractérisé en ce que**, pendant l'étape a), on mélange en chauffant dans un mélangeur de l'eau, de l'acide aminé et un sel métallique hydrosoluble jusqu'à atteindre une température de 80°C pendant au moins 15 minutes.

11. Procédé selon la revendication 10, **caractérisé en ce qu'**on introduit l'eau et on la chauffe en premier dans le mélangeur, puis on ajoute l'acide aminé et le sel métallique, et on agite et on chauffe le mélange jusqu'à atteindre une température de 80°C pendant au moins 15 minutes.

12. Procédé selon l'une quelconque des revendications 1 à 11, **caractérisé en ce que**, pour réaliser du glycinate de fer anhydre, on utilise comme acide aminé la glycine, on utilise comme sel métallique du sulfate de fer II heptahydrate, et le taux d'humidité est d'environ 6 %.

13. Procédé selon l'une quelconque des revendications 1 à 11, **caractérisé en ce que**, pour réaliser du glycinate de cuivre dihydrate, on utilise comme acide aminé la glycine, on utilise comme sel métallique du sulfate de cuivre pentahydrate, et le taux d'humidité est d'environ 13 %.

14. Procédé selon l'une quelconque des revendications 1 à 11, **caractérisé en ce que**, pour réaliser du glycinate de zinc anhydre, on utilise comme acide aminé la glycine, on utilise comme sel métallique du sulfate de zinc monohydrate, et le taux d'humidité est d'environ 2 %.

15. Procédé selon l'une quelconque des revendications 1 à 11, **caractérisé en ce que**, pour réaliser du glycinate de zinc dihydrate, on utilise comme acide aminé la glycine, on utilise comme sel métallique du sulfate de zinc monohydrate, et le taux d'humidité est d'environ 13 %.

16. Procédé selon l'une quelconque des revendications 1 à 11, **caractérisé en ce que**, pour réaliser du glycinate de zinc trihydrate, on utilise comme acide aminé la glycine, on utilise comme sel métallique du sulfate de zinc monohydrate, et le taux d'humidité est d'environ 17 %.

17. Dispositif pour la mise en oeuvre du procédé selon l'une quelconque des revendications 1 à 16, **caractérisé en ce qu'**il comprend :
- un mélangeur (50) et des moyens de chauffe (52) et de régulation de température pour produire une solution aqueuse homogène de complexe organométallique à température appropriée,
- un système de pulvérisation en lit fluidisé (7), avec une enceinte (10) et des moyens (8) de création d'un flux d'air de suspension ascendant (30) dans l'enceinte (10), et avec des moyens (16, 16a, 22) pour recevoir du mélangeur (50) un débit approprié de ladite solution aqueuse de complexe organométallique et pour le pulvériser dans l'enceinte (10),
- des moyens d'extraction (60, 61) pour extraire du lit fluidisé (140) les grains de poudre ayant une taille satisfaisante et un taux d'humidité satisfaisant.

18. Dispositif selon la revendication 17, **caractérisé en ce que** les moyens d'extraction comprennent un dispositif de sélection (61) pour prélever des grains de poudre dans le lit fluidisé (140), des moyens (62, 64, 64a) pour sélectionner et évacuer en sortie parmi les grains de poudre prélevés ceux qui ont une taille satisfaisante, et des moyens (63) pour recycler dans le lit fluidisé (140) les grains de poudre non sélectionnés.

19. Complexe organométallique cristallin en poudre à base d'acides aminés hydrosolubles, **caractérisé en ce que** les grains de poudre ont tous une taille supérieure à 25 µm environ.

20. Complexe organométallique cristallin en poudre selon la revendication 19, **caractérisé en ce que** les grains de poudre ont une taille comprise entre 100 et 800 µm environ, avantageusement entre 200 et 400 µm environ.

21. Complexe organométallique cristallin en poudre selon l'une des revendications 19 ou 20, **caractérisé en ce que** les grains de poudre ont un taux d'humidité inférieur à 20 % environ, avantageusement inférieur à 6 % environ.

22. Complexe organométallique cristallin en poudre selon l'une quelconque des revendications 19 à 21, **caractérisé en ce que** les grains de poudre ont une structure de grain issu d'un lit fluidisé, c'est-à-dire une structure stratifiée compacte.

## Claims

1. Process for producing a crystalline organometallic complex in powder form from watersoluble amino acids, comprising the steps of:
a) preparing an aqueous solution of the organometallic complex,
b) granulating and drying said solution by spraying in a fluidized bed by circulation of air,
c) extracting the powder grains from the fluidized bed when they have reached a size greater than a predetermined lower size limit and a moisture content below a predetermined maximum moisture content.

2. Process according to claim 1, **characterized in that** said predetermined lower size limit is about 25 µm.

3. Process according to claim 1, **characterized in that** said predetermined lower size limit is about 80 µm.

4. Process according to any one of claims 1 to 3, **characterized in that**, during the step of granulation and drying in a fluidized bed, the temperature and flow rate parameters of the aqueous solution and of the air are chosen so as to obtain, at the end of the step, powder grains having a predetermined moisture content.

5. Process according to any one of claims 1 to 4, **characterized in that** the maximum moisture content is between 2% and 20% by weight.

6. Process according to any one of claims 1 to 5, **characterized in that**:
- said solution is granulated and dried continuously by spraying in an air-fluidized bed,
- the temperature and flow rate parameters of the aqueous solution and of the air are continuously adapted so as to obtain in the fluidized bed a moisture content below a predetermined maximum moisture content,
- the powder grains having a size greater than a predetermined lower size limit are continuously extracted from the fluidized bed.

7. Process according to claim 6, **characterized in that**, during a preliminary training step, inlet air, outlet air and inlet aqueous solution flow rates and temperatures are determined, making it possible to produce a flow of outlet powder grains having a moisture content, measured by collecting samples, that is substantially lower than a predetermined maximum moisture content, and said flow rate and temperature values are then maintained throughout the process.

8. Process according to any one of claims 1 to 5, **characterized in that**:
- a batch of material is granulated and dried by spraying said solution in a fluidized-air bed,
- after the powder grains in the fluidized bed have reached a size larger than the predetermined lower size limit, the spraying is stopped while continuing maintenance in the fluidized bed to perform drying until a moisture content below the predetermined maximum moisture content is reached,
- the mass of powder grains having this size and this moisture content is extracted.

9. Process according to any one of claims 1 to 8, **characterized in that**, during the step of preparation of the organometallic complex, the mixture temperature is maintained in the range from about 55°C to 80°C.

10. Process according to any one of claims 1 to 9, **characterized in that**, during step a), water, amino acid and a water-soluble metal salt are mixed together with heating in a mixer until a temperature of 80°C is reached, for at least 15 minutes.

11. Process according to claim 10, **characterized in that** the water is introduced and is heated first in the mixer, then the amino acid and the metal salt are added, and the mixture is stirred and heated until a temperature of 80°C is reached, for at least 15 minutes.

12. Process according to any one of claims 1 to 11, **characterized in that**, to prepare an anhydrous iron glycinate, glycine is used as amino acid, iron II sulfate heptahydrate is used as metal salt, and the moisture content is about 6%.

13. Process according to any one of claims 1 to 11, **characterized in that**, to prepare copper glycinate dihydrate, glycine is used as amino acid, copper sulfate pentahydrate is used as metal salt, and the moisture content is about 13%.

14. Process according to any one of claims 1 to 11, **characterized in that**, to prepare anhydrous zinc glycinate, glycine is used as amino acid, zinc sulfate monohydrate is used as metal salt, and the moisture content is about 2%.

15. Process according to any one of claims 1 to 11, **characterized in that**, to prepare zinc glycinate dihydrate, glycine is used as amino acid, zinc sulfate monohydrate is used as metal salt, and the moisture content is about 13%.

16. Process according to any one of claims 1 to 11, **characterized in that**, to prepare zinc glycinate trihydrate, glycine is used as amino acid, zinc sulfate monohydrate is used as metal salt, and the moisture content is about 17%.

17. Device for implementing the process according to any one of claims 1 to 16, **characterized in that** it comprises:
- a mixer (50) and heating and temperature regulation means (52) for producing a homogeneous aqueous solution of organometallic complex at a suitable temperature,
- a system of spraying in a fluidized bed (7), with a cavity (10) and means (8) for creating an ascending flow of suspending air (30) in the cavity (10), and with means (16, 16a, 22) for receiving from the mixer (50) a suitable flow of said aqueous solution of organometallic complex and for spraying it in the cavity (10),
- extraction means (60, 61) for extracting from the fluidized bed (140) the powder grains having a satisfactory size and a satisfactory moisture content.

18. Device according to claim 17, **characterized in that** the extraction means comprise a selection device (61) for collecting powder grains in the fluidized bed (140), means (62, 64, 64a) for selecting and removing at the outlet, among the collected powder grains, those that have a satisfactory size, and means (63) for recycling into the fluidized bed (140) the unselected powder grains.

19. Crystalline organometallic complex in powder form based on watersoluble amino acids, **characterized in that** the powder grains are all greater than about 25 µm in size.

20. Crystalline organometallic complex in powder form according to claim 19, **characterized in that** the powder grains are between about 100 and 800 µm in size and advantageously between about 200 and 400 µm in size.

21. Crystalline organometallic complex in powder form according to either of claims 19 and 20, **characterized in that** the powder grains have a moisture content of less than about 20% and advantageously less than about 6%.

22. Crystalline organometallic complex in powder form according to any one of claims 19 to 21, **characterized in that** the powder grains have a grain structure derived from a fluidized bed, i.e. a compact stratified structure.

## Patentansprüche

1. Verfahren zur Herstellung eines pulverförmigen, kristallinen metallorganischen Komplexes aus wasserlöslichen Aminosäuren, umfassend die Schritte:
a) Herstellen einer wäßrigen Lösung des metallorganischen Komplexes,
b) Granulieren und Trocknen der genannten Lösung durch Pulverisierung mittels Zirkulation von Luft in einem Wirbelschichtbett,
c) Fördern der Körner des Pulvers aus dem Wirbelschichtbett, wenn diese eine Größe erreicht haben, die über einem vorherbestimmten unteren Grenzwert der Größe liegt, und einen Feuchtigkeitsgehalt, der geringer ist als ein vorherbestimmter maximaler Feuchtigkeitsgehalt.

2. Verfahren nach Anspruch 1, **dadurch gekennzeichnet, daß** der genannte vorherbestimmte untere Grenzwert der Größe etwa 25µm beträgt.

3. Verfahren nach Anspruch 1, **dadurch gekennzeichnet, daß** der genannte vorherbestimmte untere Grenzwert der Größe etwa 80µm beträgt.

4. Verfahren nach einem der Ansprüche 1 bis 3, **dadurch gekennzeichnet, daß** die Temperaturparameter und die Ausflußmenge der wäßrigen Lösung und der Luft während des Granulationsschritts und des Trocknens in dem Wirbelschichtbett in der Weise gewählt werden, daß am Ende des Verfahrensschritts die Körner des Pulvers einen vorherbestimmten Feuchtigkeitsgehalt aufweisen.

5. Verfahren nach einem der Ansprüche 1 bis 4, **dadurch gekennzeichnet, daß** der maximale Feuchtigkeitsgehalt zwischen 2 Gew.-% und 20 Gew.-% beträgt.

6. Verfahren nach einem der Ansprüche 1 bis 5, **dadurch gekennzeichnet, daß**:
- die genannte Lösung zur Pulverisierung in einem Wirbelschichtbett mittels Luft kontinuierlich granuliert und getrocknet wird,
- die Temperaturparameter und die Ausflußmenge der wäßrigen Lösung und der Luft ständig angepaßt werden, um in dem Wirbelschichtbett einen Feuchtigkeitsgehalt zu erhalten, der geringer ist als ein vorherbestimmter maximaler Feuchtigkeitsgehalt,
- die Körner des Pulvers mit einer Größe, die über einem vorherbestimmten unteren Grenzwert der Größe liegt, kontinuierlich aus dem Wirbelschichtbett herausgefördert werden.

7. Verfahren nach Anspruch 6, **dadurch gekennzeichnet, daß** während eines vorhergehenden Verfahrensschrittes als erster Versuch der Fluß und die Temperaturen der einströmenden und ausströmenden Luft und der einströmenden wäßrigen Lösung bestimmt werden, um beim Ausströmen einen Körnerfluß des Pulvers zu erhalten, der einen ausgewählten Feuchtigkeitsgrad aufweist, gemessen durch die Entnahme von Proben, der geringer ist als ein vorherbestimmter maximaler Feuchtigkeitsgehalt, und daß anschließend die genannten Werte des Flusses und der Temperatur während der Dauer des Verfahrens eingehalten werden.

8. Verfahren nach einem der Ansprüche 1 bis 5, **dadurch gekennzeichnet, daß**:
- ein Anteil der Substanz durch Pulverisierung der genannten Lösung in einem Wirbelschichtbett mittels Luft kontinuierlich granuliert und getrocknet wird,
- die Pulverisierung unterbrochen wird, nachdem die Körner des Pulvers in dem Wirbelschichtbett eine Größe erreicht haben, die über einem vorherbestimmten unteren Grenzwert der Größe liegt, und daß sie zum Trocknen in dem Wirbelschichtbett gehalten werden, bis ein Feuchtigkeitsgehalt erreicht ist, der geringer ist als ein vorherbestimmter maximaler Feuchtigkeitsgehalt,
- die Menge der Körner des Pulvers, welche die Größe und den Feuchtigkeitsgehalt aufweisen, gewonnen werden.

9. Verfahren nach einem der Ansprüche 1 bis 8, **dadurch gekennzeichnet, daß** während des Verfahrensschritts der Herstellung des organometallischen Komplexes eine Temperatur der Mischung in dem Bereich von etwa 55°C bis 80°C aufrechterhalten wird.

10. Verfahren nach einem der Ansprüche 1 bis 9, **dadurch gekennzeichnet, daß** während des Verfahrensschrittes a) in einem Wassermischer unter Erwärmen eine Aminosäure und ein wasserlösliches Metallsalz zumindest 15 Minuten lang gemischt werden, bis eine Temperatur von 80°C erreicht ist.

11. Verfahren nach Anspruch 10, **dadurch gekennzeichnet, daß** Wasser hinzugegeben und dieses zunächst in dem Mischer erhitzt wird, daß dann die Aminosäure und das Metallsalz hinzugefügt werden und die Mischung zumindest 15 Minuten lang gerührt und erwärmt wird, bis eine Temperatur von 80°C erreicht ist.

12. Verfahren nach einem der Ansprüche 1 bis 11, **dadurch gekennzeichnet, daß** zur Herstellung von wasserfreiem Eisenglycinat als Aminosäure Glycin und als Metallsalz Eisen(II)sulfat-Heptahydrat eingesetzt wird, und daß der Feuchtigkeitsgehalt etwa 6% beträgt.

13. Verfahren nach einem der Ansprüche 1 bis 11, **dadurch gekennzeichnet, daß** zur Herstellung von Kupferglycinat-Dihydrat als Aminosäure Glycin und als Metallsalz Kupfersulfat-Pentahydrat eingesetzt wird, und daß der Feuchtigkeitsgehalt etwa 13% beträgt.

14. Verfahren nach einem der Ansprüche 1 bis 11, **dadurch gekennzeichnet, daß** zur Herstellung von wasserfreiem Zinkglycinat als Aminosäure Glycin und als Metallsalz Zinksulfat-Monohydrat eingesetzt wird, und daß der Feuchtigkeitsgehalt etwa 2% beträgt.

15. Verfahren nach einem der Ansprüche 1 bis 11, **dadurch gekennzeichnet, daß** zur Herstellung von Zinkglycinat-Dihydrat als Aminosäure Glycin und als Metallsalz Zinksulfat-Monohydrat eingesetzt wird, und daß der Feuchtigkeitsgehalt etwa 13% beträgt.

16. Verfahren nach einem der Ansprüche 1 bis 11, **dadurch gekennzeichnet, daß** zur Herstellung von Zinkglycinat-Trihydrat als Aminosäure Glycin und als Metallsalz Zinksulfat-Monohydrat eingesetzt wird, und daß der Feuchtigkeitsgehalt etwa 17% beträgt.

17. Vorrichtung zur Durchführung des Verfahrens nach einem der Ansprüche 1 bis 16, **gekennzeichnet durch**:
- einen Mischer (50) und Mittel zum Heizen (52) und zur Regelung der Temperatur, um eine homogene wäßrige Lösung des metallorganischen Komplexes bei einer geeigneten Temperatur herzustellen,
- eine Wirbelschicht-Sprühanlage (7) mit einem Raum (10) und Mitteln (8), um eine unterbrechbare Luftströmung (30) zu erzeugen, die in dem Raum (10) aufsteigt, und mit Mitteln (16, 16a, 22) zur Aufnahme einer geeigneten Ausflußmenge der genannten wäßrigen Lösung des metallorganischen Komplexes aus dem Mischer (50) und um diesen in dem Raum (10) zu pulverisieren,
- Fördermittel (60, 61), um die Körner des Pulvers, welche eine zufriedenstellende Größe und einen zufriedenstellenden Feuchtigkeitsgrad aufweisen, aus dem Wirbelschichtbett (140) zu fördern.

18. Vorrichtung nach Anspruch 17, **dadurch gekennzeichnet, daß** die Fördermittel eine Auswahlvorrichtung (61) umfassen, um die Körner des Pulvers in dem Wirbelschichtbett (140) zu entnehmen, Mittel (62, 64, 64a) um am Auslaß zwischen den entnommenen Körnern des Pulvers auszuwählen und die abzuführen, welche eine zufriedenstellende Größe aufweisen, und Mittel (63) um die Körner des Pulvers zu dem Wirbelschichtbett (140) zurückzuführen, die nicht ausgewählt wurden.

19. Kristalliner, pulverförmiger metallorganischer Komplex basiert auf wasserlöslichen Aminosäuren, **dadurch gekennzeichnet, daß** alle Körner des Pulvers größer sind als etwa 25µm.

20. Kristalliner, pulverförmiger metallorganischer Komplex nach Anspruch 19, **dadurch gekennzeichnet, daß** alle Körner des Pulvers eine Größe zwischen etwa 100 und 800 µm aufweisen, vorzugsweise zwischen etwa 200 und 400µm.

21. Kristalliner, pulverförmiger metallorganischer Komplex nach Anspruch 19 oder 20, **dadurch gekennzeichnet, daß** die Körner des Pulvers einen Feuchtigkeitsgrad unter etwa 20%, vorzugsweise unter etwa 6% aufweisen.

22. Kristalliner, pulverförmiger metallorganischer Komplex nach einem der Ansprüche 19 bis 21, **dadurch gekennzeichnet, daß** die Körner des Pulvers eine Kornstruktur aufweisen, die zurückzuführen ist auf eine Wirbelschicht, d.h. eine kompakte Schichtstruktur.
